# EUROPEAN PATENT APPLICATION

(11) **EP 1 099 441 A2**
(43) Date of publication of application: **16.05.2001**
(21) Application number: 00309753.2
(22) Date of filing: 03.11.2000
(51) Int. Cl.: A61K 31/472, A61K 31/4725, A61K 31/445, A61K 31/495, A61P 3/00, A61P 3/04

(54) **Compositions containing apo B secretion/MTP inhibitors and anti-obesity agents and use thereof**

(30) Priority: 10.11.1999 US 164780 P
(71) Applicant: Pfizer Products Inc., Groton, Connecticut 06340 (US)
(72) Inventor: Morgan, Bradley Paul, Groton, Connecticut 06340 (US); Swick, Andrew Gordon, Groton, Connecticut 06340 (US)
(74) Representative: Hayles, James Richard

(57) **Abstract**

The invention provides methods and pharmaceutical compositions useful in treating obesity in an animal, preferably a mammal including a human subject, a companion animal, or livestock, using an apo B secretion/MTP inhibitor and an anti-obesity agent. The invention further relates to a kit comprising an amount of an apolipoprotein B secretion/microsomal triglyceride transfer protein inhibitor and a pharmaceutically acceptable carrier, vehicle or diluent in a first unit dosage form; an amount of an anti-obesity agent and a pharmaceutically acceptable carrier, vehicle or diluent in a second unit dosage form; and a container.

## Description

### BACKGROUND OF THE INVENTION

Conventional therapeutic approaches to the treatment of obesity in animals, including humans, companion animals, and livestock have traditionally focused on the regulation of energy intake. Unfortunately, there is now a growing awareness that, while moderation of caloric intake is initially effective in reducing body weight, such regimens are not particularly effective over the long-term. In response thereto, alternative strategies requiring less rigorous observation of caloric consumption have been developed. Exemplary alternative regimens include the use of anorectic agents, reduction of dietary fat absorption, and the like.

The present invention is directed to methods and pharmaceutical compositions useful in the treatment of obesity in an animal, preferably a mammal including a human subject, a companion animal, or livestock, using an apolipoprotein B (apo B) secretion/microsomal triglyceride transfer protein (MTP) inhibitor, and an anti-obesity agent.

Microsomal triglyceride transfer protein catalyzes the transport of triglyceride, cholesteryl ester and phospolipids and is strongly implicated as a mediator in the assembly of apo B-containing lipoproteins, biomolecules which contribute to the formation of atherosclerotic lesions. Specifically, the subcellular (lumen of the endoplasmic reticulum) and tissue distribution (liver and intestine) of MTP have led to speculation that it plays a role in the assembly of plasma lipoproteins, as these are known sites of plasma lipoprotein assembly. The ability of MTP to catalyze the transport of triglyceride between membranes is consistent with this hypothesis and suggests that MTP may catalyze the transport of triglyceride from its site of synthesis in the endoplasmic reticulum membrane to nascent lipoprotein particles within the lumen of the endoplasmic reticulum.

Compounds that inhibit apo B secretion and/or inhibit MTP are accordingly useful in the treatment of diseases and conditions in which, by inhibiting apo B secretion and/or MTP, serum cholesterol and triglyceride levels may be reduced. Such conditions may include, for example, hypercholesterolemia, hypertriglyceridemia, pancreatitis, atherosclerosis, diabetes and the like. For detailed discussions see, for example, Wetterau et al., Science, 258, 999-1001 (1992) and Wetterau et al., Biochem. Biophys. Acta., 875, 610-617 (1986).

Specific examples of compounds having utility as apo B secretion/MTP inhibitors are disclosed in European Patent Application Publication Nos. EP 0 584 446 and EP 0 643 057, the latter of which discloses certain compounds of the generic formulae 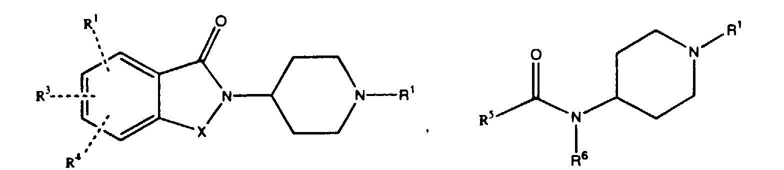 and 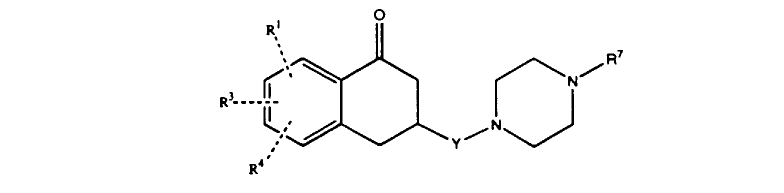 which have utility as inhibitors of MTP.

Furthermore, commonly assigned PCT International Application Publication Nos. WO 96/40640 and WO 98/23593, each of which designate, *inter alia,* the United States, disclose certain tetrahydroisoquinolines having utility as apo B secretion/MTP inhibitors. The disclosures of each of the aforementioned PCT International Application Publication Nos. WO 96/40640 and WO 98/23593 are incorporated herein by reference. Additional apo B secretion/MTP inhibitors useful in the practice of the instant invention are known, or will be apparent in light of this disclosure, to one of ordinary skill in the art.

### SUMMARY OF THE INVENTION

The invention provides methods and pharmaceutical compositions useful in the treatment of obesity in an animal, preferably a mammal including a human subject, a companion animal, or livestock, which methods comprise administering to an animal in need of such treatment, obesity-treating amounts of an apolipoprotein B (apo B) secretion/microsomal triglyceride transfer protein (MTP) inhibitor, and an anti-obesity agent. Preferably, the apo B secretion/MTP inhibitor and the anti-obesity agent are administered in synergistic therapeutically effective amounts.

The apo B secretion/MTP inhibitor is a compound preferably selected from the group consisting of:
(i) a compound of formula (I) 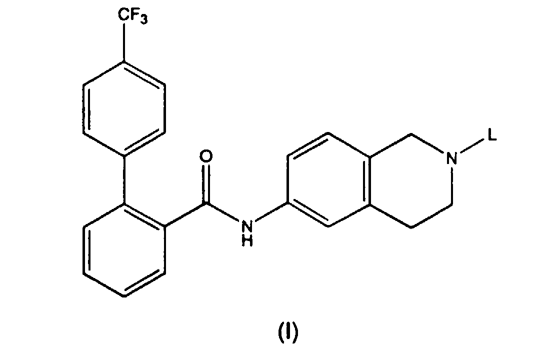 where L is as defined hereinbelow, the stereoisomers and hydrates thereof, and the pharmaceutically acceptable salts of the compounds, stereoisomers, and hydrates;
(ii) the compound BMS-197636, also known as 9-[4-[4-(2,3-dihydro-1-oxo-1H-isoindol-2-yl)-1-piperidinyl]butyl]-N-propyl-9H-fluorene-9-carboxamide, and the pharmaceutically acceptable salts thereof;
(iii) the compound BMS-200150, also known as 2-[1-(3,3-diphenylpropyl)-4-piperidinyl]-2,3-dihydro-1H-isoindol-1-one, and the pharmaceutically acceptable salts thereof; and
(iv) the compound BMS-201038, also known as 9-[4-(4-[2-(4-trifluoromethylphenyl)benzoylamino]piperidin-1-yl)butyl]-N-2,2,2-trifluoroethyl)-9H-fluorene-9-carboxamide, and the pharmaceutically acceptable salts thereof.

Especially preferred compounds of formula (I) are selected from the group consisting of 4'-trifluoromethyl-biphenyl-2-carboxylic acid-(2-butyl-1,2,3,4-tetrahydroisoquinolin-6-yl)-amide and 4'-trifluoromethyl-biphenyl-2-carboxylic acid-[2-(2-acetylaminoethyl)-1,2,3,4-tetrahydroisoquinolin-6-yl amide, the hydrates thereof, and the pharmaceutically acceptable salts of the compounds, and the hydrates.

Preferably, the anti-obesity agent is selected from the group consisting of a β₃-adrenergic receptor agonist, a cholecystokinin-A agonist, a monoamine reuptake inhibitor, a sympathomimetic agent, a serotoninergic agent, a dopamine agonist, a melanocyte-stimulating hormone receptor agonist or mimetic, a melanocyte-stimulating hormone receptor analog, a cannabinoid receptor antagonist, a melanin concentrating hormone antagonist, leptin, a leptin analog, a leptin receptor agonist, a galanin antagonist, a lipase inhibitor, a bombesin agonist, a Neuropeptide-Y antagonist, a thyromimetic agent, dehydroepiandrosterone or an analog thereof, a glucocorticoid receptor agonist or antagonist, an orexin receptor antagonist, a urocortin binding protein antagonist, a glucagon-like peptide-1 receptor agonist, a ciliary neurotrophic factor, and a human agouti-related protein antagonist.

Particularly preferred anti-obesity agents include those compounds selected from the group consisting of sibutramine, orlistat, fenfluramine, dexfenfluramine, bromocriptine, phentermine, ephedrine, leptin, phenylpropanolamine, pseudoephedrine, {4-[2-(2-[6-aminopyridin-3-yl]-2(R)-hydroxyethylamino)ethoxy]phenyl}acetic acid, {4-[2-(2-[6-aminopyridin-3-yl]-2(R)-hydroxyethylamino)ethoxy]phenyl}benzoic acid, {4-[2-(2-[6-aminopyridin-3-yl]-2(R)-hydroxyethylamino)ethoxy]phenyl}propionic acid, and {4-[2-(2-[6-aminopyridin-3-yl]-2(R)-hydroxyethylamino)ethoxy]phenoxy}acetic acid.

Tne invention further provides pharmaceutical compositions comprising amounts of an apo B secretion/MTP inhibitor, preferably an apo B secretion/MTP inhibitor shown and described hereinabove, an anti-obesity agent and, preferably, a pharmaceutically acceptable carrier, vehicle or diluent, and methods of using such compositions in treating obesity in an animal, preferably a mammal including a human subject, a companion animal, or livestock, which comprise administering to an animal in need of such treatment, an obesity treating amount of such composition.

The invention still further provides a kit comprising an amount of an apolipoprotein B secretion/microsomal triglyceride transfer protein inhibitor and a pharmaceutically acceptable carrier, vehicle or diluent in a first unit dosage form; an amount of an anti-obesity agent and a pharmaceutically acceptable carrier, vehicle or diluent in a second unit dosage form; and a container.

### DETAILED DESCRIPTION OF THE INVENTION

The instant invention provides methods and pharmaceutical compositions useful for treating obesity in an animal, preferably a mammal including a human subject, a companion animal, or livestock, which methods comprise administering to an animal in need of such treatment obesity-treating amounts of an apo B secretion/MTP inhibitor, and an anti-obesity agent. Preferably, the apo B secretion/MTP inhibitor and the anti-obesity agent are administered in synergistic therapeutically effective amounts.

Although any apo B secretion/MTP inhibitor may be employed in the methods and compositions of the instant invention, the apo B secretion/MTP inhibitor is preferably a compound selected from the group consisting of:
(i) a compound of formula (I) 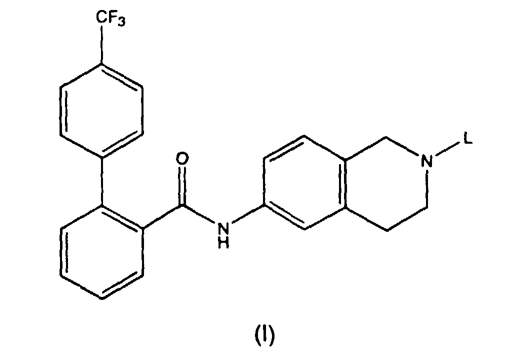 the stereoisomers and hydrates thereof, and the pharmaceutically acceptable salts of the compounds, stereoisomers and hydrates, wherein L represents:
   (A) X-Y-Z, wherein:
      X is a moiety selected from the group consisting of CH₂, CO, CS, or SO₂;
      Y is a moiety selected from the group consisting of a direct link, aliphatic hydrocarbylene radicals having up to 20 carbon atoms, which radical may be monosubstituted by hydroxy, (C₁-C₁₀)alkoxy, (C₁-C₁₀)acyl, (C₁-C₁₀)acyloxy, or (C₆-C₁₀)aryl, NH, and O, provided that if X is CH₂, Y is a direct link; and
      Z is a moiety selected from the group consisting of:
         (1) hydrogen, halogen, cyano,
         (2) hydroxy, (C₁-C₁₀)alkoxy, (C₁-C₁₀)alkylthio, (C₁-C₁₀)acyl, thiophenylcarbonyl, (C₁-C₁₀)alkoxycarbonyl,
         (3) (C₁-C₁₀)alkylamino, di(C₁-C₁₀)alkylamino, (C₆-C₁₀)aryl(C₁-C₁₀)alkylamino, provided that Y is not O or NH,
         (4) unsubstituted vinyl, (C₆-C₁₀)aryl, (C₃-C₈)cycloalkyl and fused benz derivatives thereof, (C₇-C₁₀)Polycycloalkyl, (C₄-C₈)cycloalkenyl, (C₇-C₁₀)polycycloalkenyl,
         (5) (C₆-C₁₀)aryloxy, (C₆-C₁₀)arylthio, (C₆-C₁₀)aryl(C₁-C₁₀)alkoxy, (C₆-C₁₀)aryl(C₁-C₁₀)alkylthio, (C₃-C₈)cycloalkyloxy, (C₄-C₈)cycloalkenyloxy,
         (6) heterocyclyl selected from the group consisting of monocyclic radicals and fused polycyclic radicals, wherein said radicals contain a total of from 5 to 14 ring atoms, wherein said radicals contain a total of from 1 to 4 ring heteroatoms independently selected from oxygen, nitrogen, and sulfur, and wherein the individual rings of said radicals may be independently saturated, partially unsaturated, or aromatic, provided that if X is CH₂, Z is H or is selected from groups (4) and (6),
         wherein, when Z contains one or more rings, said rings may each independently bear 0 to 4 substituents independently selected from halo, hydroxy, cyano, nitro, oxo, thioxo, aminosulfonyl, phenyl, phenoxy, phenylthio, halophenylthio, benzyl, benzyloxy, (C₁-C₁₀)alkyl, (C₁-C₁₀)alkoxy, (C₁-C₁₀)alkoxycarbonyl, (C₁-C₁₀)alkylthio, (C₁-C₁₀)alkylamino, (C₁-C₁₀)alkylaminocarbonyl, di(C₁-C₁₀)alkylamino, di(C₁-C₁₀)alkylaminocarbonyl, di(C₁-C₁₀)alkylamino(C₁-C₁₀)alkoxy, (C₁-C₃)perfluoroalkyl, (C₁-C₃)perfluoroalkoxy, (C₁-C₁₀)acyl, (C₁-C₁₀)acyloxy, (C₁-C₁₀)acyloxy(C₁-C₁₀)alkyl, and pyrrolidinyl; or
   (B) G, wherein G is selected from the group consisting of:
      (a) a phenyl or heterocyclic ring wherein said heterocyclic ring contains a total of from 3 to 14 ring atoms, wherein said heterocyclic ring incorporates a total of from 1 to 4 ring heteroatoms selected independently from oxygen, nitrogen, and sulfur, wherein the individual rings of said heterocyclic ring may be independently saturated, partially saturated or aromatic, and wherein each of said phenyl or heterocyclic rings may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acyloxy, (C₁-C₆)acylamino and (C₁-C₆)perfluoroacylamino;
      (b) -CH₂CN,
      (c) 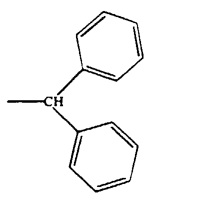
      (d) (C₂-C₁₂)alkyl or (C₂-C₁₂)perfluoroalkyl wherein each of said (C₂-C₁₂)alkyl and (C₂-C₁₂)perfluoroalkyl is substituted optionally with from 1-3 substituents selected independently from:
         (1) phenyl, halogen, nitro, cyano, hydroxy, -NR¹R², -OCOR³, (C₁-C₄)alkoxy, (C₁-C₄)perfluoroalkoxy, (C₁-C₄)thioalkoxy or (C₁-C₄)perfluorothioalkoxy,
            where R¹ and R² in the definition of -NR¹R² are each selected independently from hydrogen, formyl, phenyl, benzyl, benzoyl, (C₃-C₈)cycloalkyl, (C₃-C₈)cycloalkenyl, (C₁-C₄)alkyl, (C₁-C₄)perfluoroalkyl, (C₁-C₁₀)alkoxycarbonyl, (C₁-C₆)acyl, (C₁-C₆)perfluoroacyl, aminocarbonyl, (C₁-C₁₀)alkylaminocarbonyl, di(C₁-C₁₀)alkylaminocarbonyl, aminosulfonyl, (C₁-C₄)alkylaminosulfonyl, di(C₁-C₄)alkylaminosulfonyl, (C₁-C₄)perfluoroalkylaminosulfonyl, di(C₁-C₄)perfluoroalkylaminosulfonyl, (C₁-C₄)alkylsulfonyl, and (C₁-C₄)perfluoroalkylsulfonyl,
            or where R¹ and R², taken together with the nitrogen atom to which they are attached, form a saturated, partially-saturated or aromatic heterocyclic ring, wherein said heterocyclic ring contains a total of from 3 to 14 ring atoms and incorporates optionally an additional 1 to 4 ring heteroatoms selected independently from oxygen, nitrogen and sulfur, wherein said heterocyclic ring may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, and (C₁-C₁₀)acyloxy,
            where R³ in the definition of -OCOR³ is selected from -NR¹R², phenyl, (C₁-C₁₀)alkyl, (C₁-C₄)perfluoroalkyl, (C₁-C₆)alkoxy and (C₁-C₆)perfluoroalkoxy,
         (2) (C₃-C₈)cycloalkyl or (C₃-C₈)cycloalkenyl wherein each of said (C₃-C₈)cycloalkyl and (C₃-C₈)cycloalkenyl may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, (C₁-C₁₀)perfluoroacylamino, (C₁-C₁₀)acyloxy, and
         (3) a saturated, partially-saturated or aromatic heterocyclic ring containing a total of from 3 to 14 ring atoms, wherein said heterocyclic ring incorporates a total of from 1 to 4 ring heteroatoms selected independently from oxygen, nitrogen and sulfur, wherein said heterocyclic ring may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, (C₁-C₁₀)perfluoroacylamino, (C₁-C₁₀)acyloxy;
   (e) (C₃-C₈)cycloalkyl or (C₃-C₈)cycloalkenyl wherein each of said (C₃-C₈)cycloalkyl and (C₃-C₈)cycloalkenyl may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkythio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, (C₁-C₁₀)perfluoroacylamino, (C₁-C₁₀)acyloxy; and
   (f) -(CH₂)ₙCOR⁴, where R⁴ in the definition of -(CH₂)ₙCOR⁴ is selected from hydroxy, phenyl, -NR¹R², (C₁-C₄)alkyl, (C₁-C₄)perfluoroalkyl, (C₁-C₄)alkoxy, (C₁-C₄)perfluoroalkoxy, (C₃-C₈)cycloalkyl, and (C₃-C₈)cycloalkenyl, where n is an integer from 1 to 4;
(ii) the compound BMS-197636, also known as 9-[4-[4-(2,3-dihydro-1-oxo-1H-isoindol-2-yl)-1-piperidinyl]butyl]-N-propyl-9H-fluorene-9-carboxamide, and the pharmaceutically acceptable salts thereof;
(iii) the compound BMS-200150, also known as 2-[1-(3,3-diphenylpropyl)-4-piperidinyl]-2,3-dihydro-1H-isoindol-1-one, and the pharmaceutically acceptable salts thereof; and
(iv) the compound BMS-201038, also known as 9-[4-(4-[2-(4-trifluoromethylphenyl)benzoylamino]piperidin-1-yl)butyl]-N-2,2,2-trifluoroethyl)-9H-fluorene-9-carboxamide, and the pharmaceutically acceptable salts thereof.

A preferred subgroup of formula (I) compounds includes those compounds selected from the group consisting of 4'-trifluoromethyl-biphenyl-2-carboxylic acid-(2-butyl-1,2,3,4-tetrahydroisoquinolin-6-yl)-amide, 4'-trifluoromethyl-biphenyl-2-carboxylic acid-[2-(2-acetylaminoethyl)-1,2,3,4-tetrahydroisoquinolin-6-yl amide, the hydrates thereof, and the pharmaceutically acceptable salts of the compounds, and the hydrates.

Tne invention further provides pharmaceutical compositions comprising amounts of an apo B secretion/MTP inhibitor, preferably an apo B secretion/MTP inhibitor shown and described hereinabove, an anti-obesity agent and, preferably, a pharmaceutically acceptable carrier, vehicle or diluent, and methods of using such compositions in treating obesity in an animal, preferably a mammal including a human subject, a companion animal, or livestock, which comprise administering to an animal in need of such treatment, an obesity treating amount of such composition.

The invention still further provides a kit comprising an amount of an apolipoprotein B secretion/microsomal triglyceride transfer protein inhibitor and a pharmaceutically acceptable carrier, vehicle or diluent in a first unit dosage form; an amount of an anti-obesity agent and a pharmaceutically acceptable carrier, vehicle or diluent in a second unit dosage form; and a container.

For purposes of this invention, an "animal" includes a warm-blooded animal of the animal kingdom possessed of a homeostatic mechanism and includes mammals and birds, preferably humans, companion animals such as dogs, cats and horses, and livestock, such as cows, pigs, and the like.

The following selected functional group definitions and examples thereof are employed throughout the instant description and appendant claims and are offered by way of illustration, and not by limitation.

The term "heterocyclyl" as employed within the definition of Z is meant to embrace any single ring or fused ring system containing at least one ring heteroatom independently selected from O, N, and S. Thus, a polycyclic fused ring system containing one or more carbocyclic fused saturated, partially unsaturated or aromatic rings (usually benz rings) is within the definition of heterocyclyl so long as the system also contains at least one fused ring which contains at least one of the aforementioned heteroatoms. As a substituent, such heterocyclyls may be attached to the remainder of the molecule from either a carbocyclic (e.g. benz) ring or from a heterocyclic ring.

The phrase "one or more rings" when employed in the definition of Z is intended to mean any (single or fused) cyclic moiety or moieties contained in Z. The rings may be carbocyclic or heterocyclic, saturated or partially unsaturated and aromatic or non-aromatic.

Reference to a fused polycyclic ring system or radical means that all rings in the system are fused.

The term "acyl" when employed in the description of a substituent refers to an aliphatic or hydrocarbon moiety attached to a carbonyl group through which the substituent bonds. Representative of such acyl moieties are acetyl, propionyl, butyryl, isobutyryl, and the like.

Reference to the terms "alkyl" and "alkoxy" includes both straight and branched chain radicals, however, it is to be understood that references to individual radicals, for example "propyl" or "propoxy", embrace only the straight chain radical, branched chain isomers such as "isopropyl" or "isopropoxy" being referred to specifically.

The term "aliphatic hydrocarbylene radical" means a divalent, open-chain organic radical containing carbon and hydrogen only. The radical serves as a linking group, denoted hereinabove as Y. The radical may be straight chain or branched and/or saturated or unsaturated, containing up to three unsaturated bonds, either double, triple or a mixture of double and triple. The two valences may be on different carbon atoms or on the same carbon atom, and thus the term "alkylidene" is subsumed under this definition. The radical will typically be classified as a (C₁-C₂₀)alkylene radical, a (C₂-C₂₀)alkenylene radical or a (C₂-C₂₀)alkynylene radical. Typically, the radical will contain 1-10 carbon atoms, although longer chains are certainly feasible and within the scope of this invention.

The term "aryl", e.g. (C₆-C₁₀)aryl, when employed in the description of a substituent means the ring or substituent is carbocyclic. Aromatic moieties which contain one or more heteroatoms are included as a subset of the term "heterocyclyl" as discussed hereinabove.

The term "halogen" employed throughout the description and appendant claims is inclusive of fluoro, chloro, bromo and iodo, unless noted otherwise.

The term "perfluoro", when used in conjunction with a specified hydrocarbon radical, is meant to include a substituent wherein the individual hydrogen atoms thereof may be substituted therefor with one or more, and preferably, from 1 to 9 fluorine atoms. Exemplary of such radicals are trifluoromethyl, pentafluoroethyl, heptafluoropropyl, and the like.

The central benz-heterocyclic ring system of formula (I), i.e. the fused bicyclic ring system attached through its single ring nitrogen to L, is referred to herein as a "1,2,3,4-tetrahydroisoquinoline" for convenience in nomenclature, and this is the convention most commonly employed when naming compounds according to the invention as 2-substituted-1,2,3,4-tetrahydroisoquinolin-6-yl amides. It is noted that, less frequently when named as a substituent in a compound, this central ring system is also denoted as a 6-substituted "3,4-dihydro-1H-isoquinolin-2-yl" moiety.

It will be appreciated by one of ordinary skill in the art that certain compounds of formula (I) contain an asymmetrically substituted carbon atom and accordingly may exist in, and be isolated in, both optically-active and racemic forms. Some compounds may exhibit polymorphism. It is to be understood that the instant invention encompasses any racemic, optically-active, polymorphic, stereoisomeric, or mixture thereof, form of a formula (I) compound which form possesses properties useful in the methods and pharmaceutical compositions of this invention. It is well known, or will be apparent in light of the instant disclosure, to one of ordinary skill in the art how to prepare such optically-active forms (for example, by resolution of the racemic form by recrystallization techniques, by synthesis from optically-active starting materials, by chiral synthesis or by chromatographic techniques) and how to determine the efficacy of such forms in carrying out the objectives of the present invention by the standard protocols described in detail hereinbelow.

Furthermore, one of ordinary skill in the art will recognize that certain combinations of substituents or moieties listed in this invention define compounds that will be less stable under physiological conditions (e.g., those compounds containing aminal or acetal linkages). Accordingly, such compounds are less preferred.

Alkylene radicals include those saturated hydrocarbon groups having 1-20, preferably 1-10 carbon atoms, derived by removing two hydrogen atoms from a corresponding saturated acyclic hydrocarbon. Illustrative values having 1-10 carbon atoms include straight chain radicals having the formula (CH₂)ₙ wherein n is 1 to 10, such as methylene, dimethylene, trimethylene, tetramethylene, pentamethylene, hexamethylene, heptamethylene, octamethylene, nonamethylene and so forth. Also included are alkylidene radicals such as ethylidene, propylidene, butylidene and *sec*-butylidene. Also included are branched isomers such as 1,1-dimethyldimethylene, 1,1-dimethyltetramethylene, 2,2-dimethyltrimethylene and 3,3-dimethylpentamethylene.

Alkenylene radicals include those straight or branched chain radicals having 2-20 carbon atoms, preferably 2-10 carbon atoms, derived by removal of two hydrogen atoms from a corresponding acyclic hydrocarbon group containing at least one double bond. Illustrative values for alkenylene radicals having one double bond include ethenylene (vinylene), propenylene, 1-butenylene, 2-butenylene and isobutenylene. Alkenylene radicals containing two double bonds (sometimes referred to in the art as alkadienylene radicals) include 3-methyl-2,6-heptadienylene, 2-methyl-2,4-heptadienylene, 2,8-nonadienylene, 3-methyl-2,6-octadienylene and 2,6-decadienylene. An illustrative value for an alkylene radical containing three double bonds (an alkatrienylene radical) is 9,11,13-heptadecatrienylene.

Alkynylene radicals include those straight or branched chain radicals having 2-20 carbon atoms, preferably 2-10 carbon atoms, derived by removal of two hydrogen atoms from a corresponding acylic hydrocarbon group containing at least one triple bond. Illustrative values include ethynylene, propynylene, 1-butynylene, 1-pentynylene, 1-hexynylene, 2-butynylene, 2-pentynylene, 3,3-dimethyl-1-butynylene and so forth.

The following are illustrative values for other moieties and substituents named hereinabove, which are not to be construed as limiting in any respect. It is noted that throughout the description and appendant claims, if a cyclic or polycyclic radical which can be bonded through differing ring atoms is referred to without noting a specific point of attachment, all possible points are intended, whether through a carbon atom or a trivalent nitrogen atom. As examples, reference to (unsubstituted) "naphthyl" means naphth-1-yl, and naphth-2-yl; reference to "pyridyl" means 2-, 3-, or 4-pyridyl and reference to "indolyl" means attachment or bonding through any of the 1-, 2-, 3-, 4-, 5-, 6- or 7-positions.

Illustrative values for (C₁-C₁₀)alkoxy include methoxy, ethoxy, propoxy, isopropoxy, butoxy, isobutoxy, pentoxy, hexoxy, heptoxy and so forth.

Illustrative values for (C₁-C₁₀)alkylthio include the corresponding sulfur-containing compounds of (C₁-C₁₀)alkoxy hereinabove including methylthio, ethylthio, propylthio, isopropylthio, butylthio, isobutylthio, pentylthio, hexylthio, heptylthio and so forth.

Illustrative values for (C₁-C₁₀)acyl include values for (C₁-C₁₀)alkanoyl such as formyl, acetyl, propionyl, butyryl and isobutyryl. Also included are other common cycle-containing radicals such as benzoyl.

Illustrative values for (C₁-C₁₀)acyloxy include values for (C₁-C₁₀)alkanoyloxy such as formyloxy, acetyloxy, propionyloxy, butyryloxy and isobutyryloxy. Also included are other cycle-containing radicals such as benzoyloxy.

Illustrative values for (C₁-C₁₀)alkoxycarbonyl include methoxycarbonyl, ethoxycarbonyl, propoxycarbonyl, isopropoxycarbonyl, butoxycarbonyl and isobutoxycarbonyl.

Illustrative values for (C₁-C₁₀)alkylamino include methylamino, ethylamino, propylamino, isopropylamino, butylamino and isobutylamino.

Illustrative values for di-(C₁-C₁₀)alkylamino include dimethylamino, diethylamino, dipropylamino, dibutylamino and diisobutylamino.

Illustrative values for (C₆-C₁₀)aryl(C₁-C₁₀)alkylamino are benzylamino, (1-phenylethyl)amino and (2-phenylethyl)amino.

Illustrative values for (C₆-C₁₀)aryl include phenyl and naphthyl.

Illustrative values of (C₃-C₈)cycloalkyl include cyclopropyl, cyclobutyl, cyclcopentyl, cyclohexyl and cycloheptyl.

Illustrative values for fused benz derivatives of (C₃-C₈)cycloalkyl include 1,2,3,4-tetrahydronapthalenyl, indanyl and fluorenyl.

Illustrative values of polycycloalkyl include adamantyl and 2-bicyclo[2.2.1]heptyl.

Illustrative values for (C₄-C₈)cycloalkenyl include cyclobutenyl, cyclopentenyl, cyclohexenyl and cycloheptenyl.

Illustrative values for polycycloalkenyl include bicyclo[3.1.1]hept-2-enyl.

Illustrative values for (C₆-C₁₀)aryloxy include phenoxy and naphthyloxy.

Illustrative values for (C₆-C₁₀)arylthio include phenylthio and naphthylthio.

Illustrative values for (C₆-C₁₀)aryl(C₁-C₁₀)alkoxy include benzyloxy and phenylethoxy.

Illustrative values for (C₆-C₁₀)aryl(C₁-C₁₀)alkylthio include benzylthio and phenylethylthio.

Illustrative values for (C₃-C₈)cycloalkyloxy include cyclopropyloxy, cyclobutyloxy, cyclopentyloxy, cyclohexyloxy and cycloheptyloxy.

Illustrative values for (C₄-C₈)cycloalkenyloxy include cyclobutenyloxy, cyclopentenyloxy, cyclohexenyloxy and cycloheptenyloxy.

Illustrative values for heterocyclyl substituents which are five-membered monocyclic radicals include furanyl, thienyl, pyrrolyl, pyrrolidinyl, oxazolyl, thiazolyl, imidazolyl, pyrazolyl, isoxazolyl, isothiazolyl, 1,2,3-triazolyl, 1,2,4-triazolyl, 1,3,4-thiadiazolyl and the like.

Illustrative values for heterocyclyl substituents which are six-membered monocyclic radicals include 2H- and 4H-pyranyl, pyridyl, piperidinyl, piperazinyl, pyridazinyl, pyrimidinyl, pyrazinyl, morpholinyl, thiomorpholinyl, 1,3,5-triazinyl and the like.

Illustrative values for heterocyclyl substituents which are fused benz derivatives of five-membered heterocyclic radicals include indolyl, isoindolyl, indolinyl, benzofuranyl, benzothienyl, benzimidazolyl, benzthiazolyl and carbazolyl. Illustrative values for heterocyclic substituents which are fused benz derivatives of six-membered heterocyclic radicals include quinolinyl, isoquinolinyl, quinazolinyl, phthalazinyl, phenothiazinyl, acridinyl and phenoxazinyl.

Illustrative examples for heterocyclic groups which are fused polycyclic radicals other than the fused benz systems exemplified hereinabove include purinyl and pteridinyl.

Illustrative values of (C₁-C₁₀)alkyl include methyl, ethyl, propyl, isopropyl, isobutyl, butyl, *tert*-butyl, pentyl, hexyl and the like.

Illustrative values for (C₁-C₃)perfluoroalkyl include trifluoromethyl, pentafluoroethyl and heptafluoropropyl.

Illustrative values for (C₁-C₃)perfluoroalkoxy include trifluoromethoxy, pentafluoroethoxy.

The compounds of formula (I), the hydrates and stereoisomers thereof, and the pharmaceutically acceptable salts of the compounds, stereoisomers, and hydrates, are readily prepared according to the synthetic methodologies disclosed in the aforementioned PCT International Application Publication Nos. WO 96/40640 and WO 98/23593.

The compound BMS-197636, also known as 9-[4-[4-(2,3-dihydro-1-oxc-1H-isoindol-2-yl)-1-piperidinyl]butyl]-N-propyl-9H-fluorene-9-carboxamide, and the pharmaceutically acceptable salts thereof, may be prepared as disclosed in PCT International Application Publication No. WO 96/26205, the disclosure of which is incorporated herein by reference.

The compound BMS-200150, also known as 2-[1-(3,3-diphenylpropyl)-4-piperidinyl]-2,3-dihydro-1H-isoindol-1-one, and the pharmaceutically acceptable salts thereof, may be prepared as disclosed in European Patent Application Publication No. EP 0 643 057, the disclosure of which is also incorporated herein by reference.

The compound BMS-201038, also known as 9-[4-(4-[2-(4-trifluoromethylphenyl)benzoylamino]piperidin-1-yl)butyl]-N-2,2,2-trifluoroethyl)-9H-fluorene-9-carboxamide, and the pharmaceutically acceptable salts thereof, may be prepared as disclosed in U.S. Pat. No. 5,739,135, the disclosure of which is also incorporated herein by reference.

Although any anti-obesity agent may be employed in the methods of the instant invention, generally preferred anti-obesity agents are selected from the group consisting of: a β₃-adrenergic receptor agonist, a cholecystokinin-A (CCK-A) agonist, a monoamine reuptake inhibitor (such as sibutramine), a sympathomimetic agent, a serotoninergic agent (such as dexfenfluramine or fenfluramine), a dopamine agonist (such as bromocriptine), a melanocyte-stimulating hormone receptor agonist or mimetic, a melanocyte-stimulating hormone receptor analog, a cannabinoid receptor antagonist, a melanin concentrating hormone antagonist, the OB protein (referred to hereinafter as "leptin"), a leptin analog, a leptin receptor agonist, a galanin antagonist, or a lipase inhibitor (such as orlistat). Other anti-obesity agents may include anorectic agents including, for example, a bombesin agonist, a Neuropeptide-Y antagonist, a thyromimetic agent, dehydroepiandrosterone or an analog thereof, a glucocorticoid receptor agonist or antagonist, an orexin receptor antagonist, a urocortin binding protein antagonist, a glucagon-like peptide-1 receptor agonist, a ciliary neurotrophic factor, such as Axokine, or a human agouti-related protein (referred to hereinafter as AGRP) antagonist. Other anti-obesity agents useful in the practice of the instant invention are, or will be, known in light of the disclosure herein, to one of ordinary skill in the art.

Particularly preferred anti-obesity agents useful in the methods of the invention comprise β₃-adrenergic receptor agonists, sibutramine, orlistat, fenfluramine, dexfenfluramine, bromocriptine, phentermine, ephedrine, leptin, phenylpropanolamine, and pseudoephedrine.

Particularly preferred β₃-adrenergic receptor agonists include those substituted aminopyridines disclosed in commonly assigned PCT International Application Publication No. WO 96/35671, the disclosure of which is incorporated herein by reference. Especially preferred β₃-adrenergic receptor agonists disclosed therein are selected from the group consisting of {4-[2-(2-[6-aminopyridin-3-yl]-2(R)-hydroxyethylamino)ethoxy]phenyl}acetic acid, {4-[2-(2-[6-aminopyridin-3-yl]-2(R)-hydroxyethylamino)ethoxy]phenyl}benzoic acid, {4-[2-(2-[6-aminopyridin-3-yl]-2(R)-hydroxyethylamino)ethoxy]phenyl}propionic acid, and {4-[2-(2-[6-aminopyridin-3-yl]-2(R)-hydroxyethylamino)ethoxy]phenoxy}acetic acid.

The particularly preferred anorectic agent phentermine may be prepared as described in U.S. Patent No. 2,408,345, the disclosure of which is incorporated herein by reference.

The particularly preferred monoamine reuptake inhibitor sibutramine may be prepared as described in U.S. Patent No. 4,929,629, the disclosure of which is incorporated herein by reference.

The particularly preferred lipase inhibitor orlistat may be prepared as described in U.S. Patent No. 4,598,089, the disclosure of which is incorporated herein by reference.

The particularly preferred serotoninergic agents fenfluramine and dexfenfluramine may be prepared as described in U.S. Patent No. 3,198,834, the disclosure of which is incorporated herein by reference.

The particularly preferred dopamine agonist bromocriptine may be prepared as described in U.S. Patent Nos. 3,752,814 and 3,752,888, the disclosures of which are incorporated herein by reference.

The dosage of the apo B secretion/MTP inhibitor administered will generally be dependent upon the health of the subject being treated, the extent of obesity treatment desired, the nature and kind of concurrent therapy, if any, and the frequency of treatment and nature of the effect desired. In general, apo B secretion/MTP inhibitors have been reported with representative dosage ranges being from about 0.01 mg/kg/day to about 15.0 mg/kg/day. Generally preferable dosages range from about 0.1 mg/kg/day to about 1.0 mg/kg/day. However, some variability in the general dosage range may be required depending upon the age, weight, and species of the patient, the intended route of administration, and the progress and degree of severity of the obesity being treated.

The dosage of the anti-obesity agent administered will also be generally dependent upon the health of the subject being treated, the extent of obesity treatment desired, the nature and kind of concurrent therapy, if any, and the frequency of treatment and nature of the effect desired. In general, the dosage of the anti-obesity agent is generally in the range of from about 0.001 to about 50 mg/kg body weight of the subject per day, preferably from about 0.1 to about 10 mg/kg body weight of the subject per day, administered as a single or divided dose. However, some variability in the general dosage range may also be required depending upon the age, weight, and species of the patient, the intended route of administration, and the progress and degree of severity of the obesity being treated.

When the anti-obesity agent is phentermine, the dosage of phentermine is from about 0.01 to about 10 mg/kg body weight of the subject per day, preferably from about 0.1 to about 1 mg/kg body weight of the subject per day.

When the anti-obesity agent is sibutramine, the dosage of sibutramine is from about 0.01 to about 30 mg/kg body weight of the subject per day, preferably from about 0.1 to about 1 mg/kg body weight of the subject per day.

When the anti-obesity agent is fenfluramine or dexfenfluramine, the dosage range of fenfluramine or dexfenfluramine is from about 0.01 to about 30 mg/kg body weight of the subject per day, preferably from about 0.1 to about 1 mg/kg body weight of the subject per day.

When the anti-obesity agent is bromocriptine, the dosage range of bromocriptine is from about 0.01 to about 10 mg/kg body weight of the subject per day, preferably from about 0.1 to about 10 mg/kg body weight of the subject per day.

According to the methods of the invention, when the apo B secretion/MTP inhibitors, the hydrates and stereoisomers thereof, and the pharmaceutically acceptable salts of the inhibitors, hydrates and stereoisomers, and the anti-obesity agents are administered together, such administration can be sequential in time or simultaneous with the simultaneous method being generally preferred. For sequential administration, the apo B secretion/MTP inhibitor, a hydrate or stereoisomer thereof, or a pharmaceutically acceptable salt of the inhibitor, hydrate or stereoisomer, and the anti-obesity agent can be administered in any order. It is generally preferred that such administration be oral. It is especially preferred that the administration be oral and simultaneous. However, if the subject being treated is unable to swallow, or oral absorption is otherwise impaired or undesirable, parenteral or transdermal administration will be appropriate. When the apo B secretion/MTP inhibitor, a hydrate or stereoisomer thereof, or a pharmaceutically acceptable salt of the inhibitor, hydrate or stereoisomer, and the anti-obesity agent are administered sequentially, the administration of each can be by the same method or by different methods.

According to the methods of the invention, an apo B secretion/MTP inhibitor, a hydrate or stereoisomer thereof, or a pharmaceutically acceptable salt of the inhibitor, hydrate or stereoisomer, is administered with an anti-obesity agent preferably in the form of a pharmaceutical composition comprising a pharmaceutically acceptable carrier, vehicle or diluent. Accordingly, an apo B secretion/MTP inhibitor, a hydrate or stereoisomer thereof, or a pharmaceutically acceptable salt of the inhibitor, hydrate or stereoisomer of this invention, can be administered with an anti-obesity agent, in any conventional oral, parenteral or transdermal dosage form.

Suitable pharmaceutically-acceptable carriers include inert solid fillers or diluents and sterile aqueous or organic solutions. According to the methods of the invention, the apo B secretion/MTP inhibitor, a hydrate or stereoisomer thereof, or a pharmaceutically acceptable salt of the inhibitor, hydrate or stereoisomer and the anti-obesity agent, will be present in such pharmaceutical compositions in amounts sufficient to provide the desired dosage amount in the ranges described hereinabove. Thus, for oral administration, the compounds can be combined with a suitable solid or liquid carrier, vehicle or diluent to form capsules, tablets, powders, syrups, solutions, suspensions and the like. The pharmaceutical compositions may, if desired, contain additional components such as flavorants, sweeteners, excipients and the like.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

The pharmaceutical compositions described hereinabove may also be administered parenterally. For parenteral administration the pharmaceutical compositions can be combined with sterile aqueous or organic media to form injectable solutions or suspensions. Solutions or suspensions of these pharmaceutical compositions can be prepared in water suitably mixed with a surfactant such as hydroxypropylcellulose. Dispersions can also be prepared in sesame or peanut oil, ethanol, water, polyol (e.g., glycerol, propylene glycol and liquid polyethylene glycol), suitable mixtures thereof, vegetable oils, N-methyl glucamine, polyvinylpyrrolidone and mixtures thereof in oils as well as aqueous solutions of water-soluble pharmaceutically acceptable salts of the compounds. Under ordinary conditions of storage and use, these preparations contain a preservative to prevent the growth of microorganisms. The injectable solutions prepared in this manner can then be administered intravenously, intraperitoneally, subcutaneously, or intramuscularly, with intramuscular administration being the preferred parenteral route in humans. Solutions prepared for intravenous administration are preferably rendered isotonic prior to usage.

The pharmaceutical forms suitable for injectable use include sterile aqueous solutions or dispersions and sterile powders for the preparation of sterile injectable solutions or dispersions. In all cases, the form must be sterile and must be fluid to the extent that easy syringability exists. It must be stable under the conditions of manufacture and storage and preserved against contamination by microorganisms such as bacteria and fungi.

The pharmaceutical compositions may also be administered transdermally. Suitable formulations for transdermal application include obesity treating amounts of an apo B secretion/MTP inhibitor, a hydrate or stereoisomer thereof, or a pharmaceutically acceptable salt of the inhibitor, hydrate or stereoisomer, and an anti-obesity agent, or pharmaceutical composition of the invention with a suitable transdermal carrier. Preferred transdermal carriers include absorbable pharmacologically acceptable solvents to promote and assist passage through the skin of the subject being treated. Characteristically, transdermal devices comprise the form of a bandage having a backing member, a reservoir containing the compound, optionally with carriers, optionally a rate-controlling barrier to deliver the compound to the skin of the subject being treated at a controlled and predetermined rate over a prolonged period of time and means to secure the device to the skin of the subject being treated.

Methods of preparing the various pharmaceutical compositions with a desired amount of an active ingredient are known, or will be apparent in light of this disclosure, to one of ordinary skill in the art. See, for example, Remington's Pharmaceutical Sciences, Mack Publishing Company, Easton, PA, 18th Edition (1990).

The methods of the instant invention also have utility in treating obesity in companion animals, preferably dogs and cats, and livestock, such as cows, pigs, and the like. The administration of the pharmaceutical compositions described hereinabove may be effected orally, parenterally or transdermally. An amount of a pharmaceutical composition of the invention is administered such that an effective dose is received, normally a daily dose, as set forth hereinabove.

Conveniently, the medicaments can be carried in the drinking water such that a therapeutic dosage of the agents is ingested with the daily water supply. The agents can be directly metered into drinking water, preferably in the form of a liquid, water-soluble concentrate, such as an aqeuous solution of a water-soluble salt.

For purposes of alternative convenience, the active ingredients can also be added directly to the feed of the companion animal or, alternatively, in the form of an animal feed supplement, also referred to as a premix or concentrate. A premix or concentrate of the therapeutic agent in a carrier is more commonly employed for the inclusion of the agent in the feed. Suitable carriers are liquid or solid, as desired, such as water, various meals such as alfalfa meal, soybean meal, cottonseed oil meal, linseed oil meal, corncob meal and corn meal, molasses, urea, bone meal, and various mineral mixes. A particularly effective carrier is the respective animal feed itself, i.e., a small portion of such feed. The carrier facilitates uniform distribution of the active materials in the finished feed with which the premix is blended. It is important that the compounds be thoroughly blended into the premix and, subsequently, the feed. In this respect, the agents may be dispersed or dissolved in a suitable oily vehicle such as soybean oil, corn oil, cottonseed oil, and the like, or in a volatile organic solvent and then blended with the carrier. It will be appreciated that the proportions of active materials in the concentrate are capable of wide variation since the amount of agent in the finished feed may be adjusted by blending the appropriate proportion of premix with the feed to obtain a desired level of the therapeutic agents.

High potency concentrates may be blended by the feed manufacturer with a proteinaceous carrier such as soybean oil meal and other meals, as described above, to produce concentrated supplements which are suitable for direct feeding to animals. In such instances, the animals are permitted to consume the usual diet. Alternatively, such concentrated supplements may be added directly to the feed to produce a nutritionally balanced, finished feed containing a therapeutically effective level of the compounds of the invention. The mixtures are thoroughly blended by standard procedures, such as in a twin shell blender, to insure homogeniety. If the supplement is used as a top dressing for the feed, it likewise helps to insure uniformity of distribution of the active ingredient across the top of the dressed feed.

For veterinary uses, both paste and pellet formulations may also be conveniently employed. Paste formulations can be prepared readily by dispersing the active compounds in a pharmaceutically acceptable oil such as peanut oil, sesame oil, corn oil, and the like. Similarly, pellets containing a effective amounts of the compounds of the invention can be prepared by admixing the compounds with a suitable diluent such as carbowax, carnuba wax, and the like, and a lubricant, such as magnesium or calcium stearate, can be employed to improve the pelleting process.

Since the instant invention relates to the treatment of obesity with a combination of active ingredients which may be administered separately, the invention also relates to combining separate pharmaceutical compositions in kit form. A kit, according to the invention, comprises two separate pharmaceutical compositions: a first unit dosage form comprising an apo B secretion/MTP inhibitor, preferably a compound selected from the group consisting of: (i) a compound of formula (I), a stereoisomer or hydrate thereof, or a pharmaceutically acceptable salt of said compound, stereoisomer or hydrate; (ii) the compound 9-[4-[4-(2,3-dihydro-1-oxo-1H-isoindol-2-yl)-1-piperidinyl]butyl]-N-propyl-9H-fluorene-9-carboxamide, and the pharmaceutically acceptable salts thereof; (iii) the compound 2-[1-(3,3-diphenylpropyl)-4-piperidinyl]-2,3-dihydro-1H-isoindol-1-one, and the pharmaceutically acceptable salts thereof; and (iv) the compound 9-[4-(4-[2-(4-trifluoromethylphenyl)benzoylamino]piperidin-1-yl)butyl]-N-2,2,2-trifluoroethyl)-9H-fluorene-9-carboxamide, and the pharmaceutically acceptable salts thereof, and a pharmaceutically acceptable carrier, vehicle or diluent; a second unit dosage form comprising an anti-obesity agent, and a pharmaceutically acceptable carrier, vehicle or diluent; and a container. The container is used to contain the separate pharmaceutical compositions and may comprise, for example, a divided bottle or a divided foil packet, however, the separate pharmaceutical compositions may also be contained within a single, undivided container. Normally, the kit will also include directions for the administration of the separate components. The kit form is particularly advantageous when the separate components are preferably administered in different dosage forms (e.g., oral and parenteral), are administered at different dosage levels, or when titration of the individual components of the combination is desired by the prescribing physician.

One example of such a kit comprises a so-called blister pack. Blister packs are well known in the packaging industry and are being used widely for the packaging of pharmaceutical unit dosage forms (tablets, capsules and the like). Blister packs generally comprise a sheet of relatively rigid material covered with a foil of a preferably transparent plastic material. During the packaging process recesses are formed in the plastic foil. The recesses generally conform to the size and shape of the tablets or capsules to be contained therein. Next, the tablets or capsules are placed in the recesses and the sheet of relatively rigid material is sealed against the plastic foil at the face of the foil which is opposite from the direction in which the recesses were formed. As a result, the tablets or capsules are sealed in the recesses between the plastic foil and the sheet. Preferably, the strength of the sheet is such that the tablets or capsules may be removed from the blister pack by the application of manual pressure on the recesses whereby an opening is formed in the sheet at the place of the recess. The tablet or capsule can then be removed through the formed opening.

It is further desirable to provide a memory aid on the pack, e.g., in the form of numbers or similar indicia next to the tablets or capsules whereby the indicia correspond with the days of the regimen which the dosage form so specified is to be ingested. An additional example of such a memory aid is a calendar printed on the pack, e.g., as follows "First Week, Monday, Tuesday, ... etc.... Second Week, Monday, Tuesday, . . . "etc. Other variations will be readily apparent. A "daily dose" can be a single tablet or capsule or multiple tablets or capsules to be ingested on a given day. Also, a daily dose comprising an apo B secretion/MTP inhibitor, preferably a compound selected from the group consisting of: (i) a compound of formula (I), a stereoisomer or hydrate thereof, or a pharmaceutically acceptable salt of said compound, stereoisomer or hydrate; (ii) the compound 9-[4-[4-(2,3-dihydro-1-oxo-1H-isoindol-2-yl)-1-piperidinyl]butyl]-N-propyl-9H-fluorene-9-carboxamide, and the pharmaceutically acceptable salts thereof; (iii) the compound 2-[1-(3,3-diphenylpropyl)-4-piperidinyl]-2,3-dihydro-1H-isoindol-1-one, and the pharmaceutically acceptable salts thereof; and (iv) the compound 9-[4-(4-[2-(4-trifluoromethylphenyl)benzoylamino]piperidin-1-yl)butyl]-N-2,2,2-trifluoroethyl)-9H-fluorene-9-carboxamide, and the pharmaceutically acceptable salts thereof, can consist of one tablet or capsule, while a daily dose comprising an anti-obesity agent can consist of multiple tablets or capsules, or vice versa. The memory aid should reflect this.

In another specific embodiment of the invention, a pack designed to dispense the daily doses one at a time in the order of their intended use is provided. Preferably, the pack is equipped with a memory aid, so as to further facilitate compliance with the dosage regimen. An example of such a memory aid is a mechanical counter which indicates the number of daily doses to be dispensed. Another example of such a memory aid is a battery-powered microchip memory coupled with a liquid crystal readout, or audible reminder signal which, for example, reads out the date that the last daily dose has been taken and/or reminds the patient when the next dose is to be taken.

### EXPERIMENTAL

The utility of the apo B secretion/MTP inhibitor - anti-obesity agent combination in the treatment of obesity is demonstrated according to the following protocol utilizing rats as test subjects.

The apo B secretion/MTP inhibitor and the anti-obesity agent test compounds are administered by oral gavage at a dose of from about 0.1 to about 50 mg/kg for each compound for a period of up to 28 days. Body weight of the test subjects is monitored by weighing the test subjects daily. In addition, body composition, i.e. percent fat mass vs. lean mass is determined by carcass analysis or duel x-ray absorptrometry (DEA) scanning.

The use of this method for assessment of synergy in the treatment of obesity is deemed appropriate where a quantitative dose-response curve for each individual drug exists. In this instance, a synergistic response is greater than an additive quantitative response obtained with a combination of two agents compared to the same biological response at a particular dose based on the respective single agent dose-response curve.

## Claims

1. Use of an apolipoprotein B secretion/microsomal triglyceride transfer protein inhibitor and an anti-obesity agent in the manufacture of a medicament for treating obesity.

2. Use according to claim 1, wherein said apolipoprotein B secretion/microsomal triglyceride transfer protein inhibitor and said anti-obesity agent are present in synergistic therapeutically effective amounts.

3. Use according to claim 1 or claim 2, wherein said apolipoprotein B secretion/microsomal triglyceride transfer protein inhibitor is a compound selected from the group consisting of:
(i) a compound of formula (I) 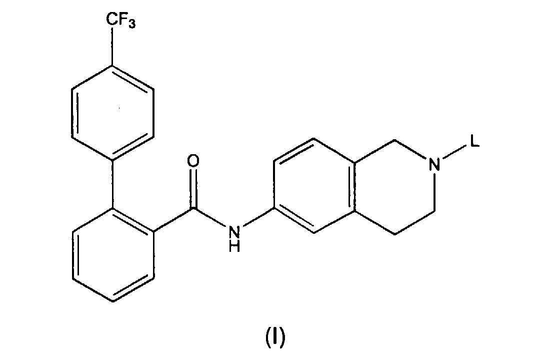 the stereoisomers and hydrates thereof, and the pharmaceutically acceptable salts of said compounds, stereoisomers and hydrates, wherein L represents:
X-Y-Z, wherein:
X is a moiety selected from the group consisting of CH₂, CO, CS, or SO₂;
Y is a moiety selected from the group consisting of a direct link, aliphatic hydrocarbylene radicals having up to 20 carbon atoms, which radical may be monosubstituted by hydroxy, (C₁-C₁₀)alkoxy, (C₁-C₁₀)acyl, (C₁-C₁₀)acyloxy, or (C₆-C₁₀)aryl, NH, and O, provided that if X is CH₂, Y is a direct link; and
Z is a moiety selected from the group consisting of:
(1) hydrogen, halogen, cyano,
(2) hydroxy, (C₁-C₁₀)alkoxy, (C₁-C₁₀)alkylthio, (C₁-C₁₀)acyl, thiophenylcarbonyl, (C₁-C₁₀)alkoxycarbonyl,
(3) (C₁-C₁₀)alkylamino, di(C₁-C₁₀)alkylamino, (C₆-C₁₀)aryl(C₁-C₁₀)alkylamino, provided that Y is not O or NH,
(4) unsubstituted vinyl, (C₆-C₁₀)aryl, (C₃-C₈)cycloalkyl and fused benz derivatives thereof, (C₇-C₁₀)polycycloalkyl, (C₄-C₈)cycloalkenyl, (C₇-C₁₀)polycycloalkenyl,
(5) (C₆-C₁₀)aryloxy, (C₆-C₁₀)arylthio, (C₆-C₁₀)aryl(C₁-C₁₀)alkoxy, (C₆-C₁₀)aryl(C₁-C₁₀)alkylthio, (C₃-C₈)cycloalkyloxy, (C₄-C₈)cycloalkenyloxy,
(6) heterocyclyl selected from the group consisting of monocyclic radicals and fused polycyclic radicals, wherein said radicals contain a total of from 5 to 14 ring atoms, wherein said radicals contain a total of from 1 to 4 ring heteroatoms independently selected from oxygen, nitrogen, and sulfur, and wherein the individual rings of said radicals may be independently saturated, partially unsaturated, or aromatic, provided that if X is CH2, Z is H or is selected from groups (4) and (6),
wherein, when Z contains one or more rings, said rings may each independently bear 0 to 4 substituents independently selected from halo, hydroxy, cyano, nitro, oxo, thioxo, aminosulfonyl, phenyl, phenoxy, phenylthio, halophenylthio, benzyl, benzyloxy, (C₁-C₁₀)alkyl, (C₁-C₁₀)alkoxy, (C₁-C₁₀)alkoxycarbonyl, (C₁-C₁₀)alkylthio, (C₁-C₁₀)alkylamino, (C₁-C₁₀)alkylaminocarbonyl, di(C₁-C₁₀)alkylamino, di(C₁-C₁₀)alkylaminocarbonyl, di(C₁-C₁₀)alkylamino(C₁-C₁₀)alkoxy, (C₁-C₃)perfluoroalkyl, (C₁-C₃)perfluoroalkoxy, (C₁-C₁₀)acyl, (C₁-C₁₀)acyloxy, (C₁-C₁₀)acyloxy(C₁-C₁₀)alkyl, and pyrrolidinyl; or
G, wherein G is selected from the group consisting of:
(a) a phenyl or heterocyclic ring wherein said heterocyclic ring contains a total of from 3 to 14 ring atoms, wherein said heterocyclic ring incorporates a total of from 1 to 4 ring heteroatoms selected independently from oxygen, nitrogen, and sulfur, wherein the individual rings of said heterocyclic ring may be independently saturated, partially saturated or aromatic, and wherein each of said phenyl or heterocyclic rings may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfuoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acyloxy, (C₁-C₆)acylamino and (C₁-C₆)perfuoroacylamino;
(b) -CH₂CN,
(c) 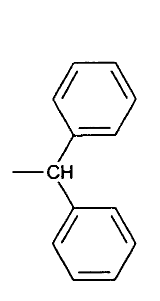
(d) (C₂-C₁₂)alkyl or (C₂-C₁₂)perfluoroalkyl wherein each of said (C₂-C₁₂)alkyl and (C₂-C₁₂)perfluoroalkyl is substituted optionally with from 1-3 substituents selected independently from:
(1) phenyl, halogen, nitro, cyano, hydroxy, -NR¹R², -OCOR³, (C₁-C₄)alkoxy, (C₁-C₄)perfluoroalkoxy, (C₁-C₄)thioalkoxy or (C₁-C₄)perfuorothioalkoxy,
where R¹ and R² in the definition of -NR¹R² are each selected independently from hydrogen, formyl, phenyl, benzyl, benzoyl, (C₃-C₈)cycloalkyl, (C₃-C₈)cycloalkenyl, (C₁-C₄)alkyl, (C₁-C₄)perfuoroalkyl, (C₁-C₁₀)alkoxycarbonyl, (C₁-C₆)acyl, (C₁-C₆)perfluoroacyl, aminocarbonyl, (C₁-C₁₀)alkylaminocarbonyl, di(C₁-C₁₀)alkylaminocarbonyl, aminosulfonyl, (C₁-C₄)alkylaminosulfonyl, di(C₁-C₄)alkylaminosulfonyl, (C₁-C₄)perfluoroalkylaminosulfonyl, di(C₁-C₄)perfuoroalkylaminosulfonyl, (C₁-C4)alkylsulfonyl, and (C₁-C₄)perfuoroalkylsulfonyl,
or where R¹ and R², taken together with the nitrogen atom to which they are attached, form a saturated, partially-saturated or aromatic heterocyclic ring, wherein said heterocyclic ring contains a total of from 3 to 14 ring atoms and incorporates optionally an additional 1 to 4 ring heteroatoms selected independently from oxygen, nitrogen and sulfur, wherein said heterocyclic ring may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfuoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, and (C₁-C₁₀)acyloxy,
where R³ in the definition of -OCOR³ is selected from -NR¹R², phenyl, (C₁-C₁₀)alkyl, (C₁-C₄)perfluoroalkyl, (C₁-C₆)alkoxy and (C₁-C₆)perfuoroalkoxy,
(2) (C₃-C₈)cycloalkyl or (C₃-C₈)cycloalkenyl wherein each of said (C₃-C₈)cycloalkyl and (C₃-C₈)cycloalkenyl may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, (C₁-C₁₀)perfuoroacylamino, (C₁-C₁₀)acyloxy, and
(3) a saturated, partially-saturated or aromatic heterocyclic ring containing a total of from 3 to 14 ring atoms, wherein said heterocyclic ring incorporates a total of from 1 to 4 ring heteroatoms selected independently from oxygen, nitrogen and sulfur, wherein said heterocyclic ring may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfuoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, (C₁-C₁₀)perfuoroacylamino, (C₁-C₁₀)acyloxy;
(e) (C₃-C₈)cycloalkyl or (C₃-C₈)cycloalkenyl wherein each of said (C₃-C₈)cycloalkyl and (C₃-C₈)cycloalkenyl may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfuoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, (C₁-C₁₀)perfluoroacylamino, (C₁-C₁₀)acyloxy; and
(f) -(CH₂)ₙCOR⁴, where R⁴ in the definition of -(CH₂)ₙCOR⁴ is selected from hydroxy, phenyl, -NR¹R², (C₁-C₄)alkyl, (C₁-C₄)perfluoroalkyl, (C₁-C₄)alkoxy, (C₁-C₄)perfluoroalkoxy, (C₃-C₈)cycloalkyl, and (C₃-C₈)cycloalkenyl, where n is an integer from 1 to 4;
(ii) the compound 9-[4-[4-(2,3-dihydro-1-oxo-1H-isoindol-2-yl)-1-piperidinyl]butyl]-N-propyl-9H-fluorene-9-carboxamide, and the pharmaceutically acceptable salts thereof;
(iii) the compound 2-[1-(3,3-diphenylpropyl)-4-piperidinyl]-2,3-dihydro-1H-isoindol-1-one, and the pharmaceutically acceptable salts thereof; and
(iv) the compound 9-[4-(4-[2-(4-trifluoromethylphenyl)benzoylamino]piperidin-1-yl)butyl]-N-2,2,2-trifluoroethyl)-9H-fluorene-9-carboxamide, and the pharmaceutically acceptable salts thereof.

4. Use according to claim 3, wherein said apolipoprotein B secretion/microsomal triglyceride transfer protein inhibitor is a compound selected from the group consisting of:
4'-trifluoromethyl-biphenyl-2-carboxylic acid-(2-butyl-1,2,3,4-tetrahydroisoquinolin-6-yl)-amide, the hydrates thereof, and the pharmaceutically acceptable salts of said compound and said hydrates;
4'-trifluoromethyl-biphenyl-2-carboxylic acid-[2-(2-acetylaminoethyl)-1,2,3,4-tetrahydroisoquinolin-6-yl amide, the hydrates thereof, and the pharmaceutically acceptable salts of said compound and said hydrates;
9-[4-[4-(2,3-dihydro-1-oxo-1H-isoindol-2-yl)-1-piperidinyl]butyl]-N-propyl-9H-fluorene-9-carboxamide, and the pharmaceutically acceptable salts thereof;
2-[1-(3,3-diphenylpropyl)-4-piperidinyl]-2,3-dihydro-1 H-isoindol-1-one, and the pharmaceutically acceptable salts thereof; and
9-[4-(4-[2-(4-trifluoromethylphenyl)benzoylamino]piperidin-1-yl)butyl]-N-2,2,2-trifluoroethyl)-9H-fluorene-9-carboxamide, and the pharmaceutically acceptable salts thereof.

5. Use according to any one of claims 1 to 4, wherein said anti-obesity agent is selected from the group consisting of a β₃-adrenergic receptor agonist, a cholecystokinin-A agonist, a monoamine reuptake inhibitor, a sympathomimetic agent, a serotoninergic agent, a dopamine agonist, a melanocyte-stimulating hormone receptor agonist or mimetic, a melanocyte-stimulating hormone receptor analog, a cannabinoid receptor antagonist, a melanin concentrating hormone antagonist, leptin, a leptin analog, a leptin receptor agonist, a galanin antagonist, a lipase inhibitor, a bombesin agonist, a Neuropeptide-Y antagonist, a thyromimetic agent, dehydroepiandrosterone or an analog thereof, a glucocorticoid receptor agonist or antagonist, an orexin receptor antagonist, a urocortin binding protein antagonist, a glucagon-like peptide-1 receptor agonist, a ciliary neurotrophic factor, and a human agouti-related protein antagonist.

6. Use according to claim 5, wherein said anti-obesity agent is selected from the group consisting of sibutramine, orlistat, fenfluramine, dexfenfluramine, bromocriptine, phentermine, ephedrine, leptin, phenylpropanolamine, pseudoephedrine, {4-[2-(2-[6-aminopyridin-3-yl]-2(R)-hydroxyethylamino)ethoxy]phenyl}acetic acid, {4-[2-(2-[6-aminopyridin-3-yl]-2(R)-hydroxyethylamino)ethoxy]phenyl}benzoic acid, {4-[2-(2-[6-aminopyridin-3-yl]-2(R)-hydroxyethylamino)ethoxy]phenyl}propionic acid, and {4-[2-(2-[6-aminopyridin-3-yl]-2(R)-hydroxyethylamino)ethoxy]phenoxy}acetic acid.

7. Use according to claim 1 or claim 2, wherein said apolipoprotein B secretion/microsomal triglyceride transfer protein inhibitor is a compound selected from the group consisting of:
(i) a compound of formula (I) 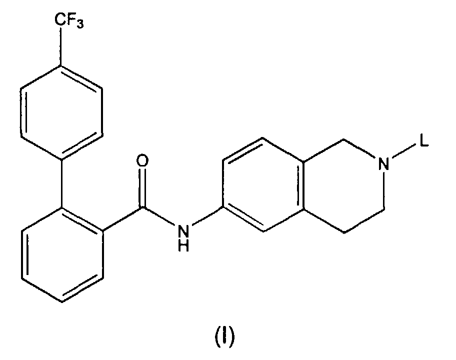 the stereoisomers and hydrates thereof, and the pharmaceutically acceptable salts of said compounds, stereoisomers and hydrates, wherein L represents:
X-Y-Z, wherein:
X is a moiety selected from the group consisting of CH₂, CO, CS, or SO₂;
Y is a moiety selected from the group consisting of a direct link, aliphatic hydrocarbylene radicals having up to 20 carbon atoms, which radical may be monosubstituted by hydroxy, (C₁-C₁₀)alkoxy, (C₁-C₁₀)acyl, (C₁-C₁₀)acyloxy, or (C₆-C₁₀)aryl, NH, and O, provided that if X is CH₂, Y is a direct link; and
Z is a moiety selected from the group consisting of:
(1) hydrogen, halogen, cyano,
(2) hydroxy, (C₁-C₁₀)alkoxy, (C₁-C₁₀)alkylthio, (C₁-C₁₀)acyl, thiophenylcarbonyl, (C₁-C₁₀)alkoxycarbonyl,
(3) (C₁-C₁₀)alkylamino, di(C₁-C₁₀)alkylamino, (C₆-C₁₀)aryl(C₁-C₁₀)alkylamino, provided that Y is not O or NH,
(4) unsubstituted vinyl, (C₆-C₁₀)aryl, (C₃-C₈)cycloalkyl and fused benz derivatives thereof, (C₇-C₁₀)polycycloalkyl, (C₄-C₈)cycloalkenyl, (C₇-C₁₀)polycycloalkenyl,
(5) (C₆-C₁₀)aryloxy, (C₆-C₁₀)arylthio, (C₆-C₁₀)aryl(C₁-C₁₀)alkoxy, (C₆-C₁₀)aryl(C₁-C₁₀)alkylthio, (C₃-C₈)cycloalkyloxy, (C₄-C₈)cycloalkenyloxy,
(6) heterocyclyl selected from the group consisting of monocyclic radicals and fused polycyclic radicals, wherein said radicals contain a total of from 5 to 14 ring atoms, wherein said radicals contain a total of from 1 to 4 ring heteroatoms independently selected from oxygen, nitrogen, and sulfur, and wherein the individual rings of said radicals may be independently saturated, partially unsaturated, or aromatic, provided that if X is CH₂, Z is H or is selected from groups (4) and (6),
wherein, when Z contains one or more rings, said rings may each independently bear 0 to 4 substituents independently selected from halo, hydroxy, cyano, nitro, oxo, thioxo, aminosulfonyl, phenyl, phenoxy, phenylthio, halophenylthio, benzyl, benzyloxy, (C₁-C₁₀)alkyl, (C₁-C₁₀)alkoxy, (C₁-C₁₀)alkoxycarbonyl, (C₁-C₁₀)alkylthio, (C₁-C₁₀)alkylamino, (C₁-C₁₀)alkylaminocarbonyl, di(C₁-C₁₀)alkylamino, di(C₁-C₁₀)alkylaminocarbonyl, di(C₁-C₁₀)alkylamino(C₁-C₁₀)alkoxy, (C₁-C₃)perfluoroalkyl, (C₁-C₃)perfluoroalkoxy, (C₁-C₁₀)acyl, (C₁-C₁₀)acyloxy, (C₁-C₁₀)acyloxy(C₁-C₁₀)alkyl, and pyrrolidinyl; or
G, wherein G is selected from the group consisting of:
(a) a phenyl or heterocyclic ring wherein said heterocyclic ring contains a total of from 3 to 14 ring atoms, wherein said heterocyclic ring incorporates a total of from 1 to 4 ring heteroatoms selected independently from oxygen, nitrogen, and sulfur, wherein the individual rings of said heterocyclic ring may be independently saturated, partially saturated or aromatic, and wherein each of said phenyl or heterocyclic rings may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acyloxy, (C₁-C₆)acylamino and (C₁-C₆)perfluoroacylamino;
(b) -CH₂CN,
(c) 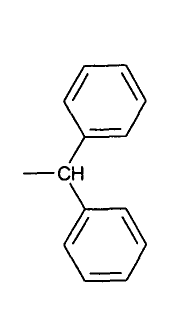
(d) (C₂-C₁₂)alkyl or (C₂-C₁₂)perfluoroalkyl wherein each of said (C₂-C₁₂)alkyl and (C₂-C₁₂)perfluoroalkyl is substituted optionally with from 1-3 substituents selected independently from:
(1) phenyl, halogen, nitro, cyano, hydroxy, -NR¹R², -OCOR³, (C₁-C₄)alkoxy, (C₁-C₄)perfluoroalkoxy, (C₁-C₄)thioalkoxy or (C₁-C₄)perfluorothioalkoxy,
where R¹ and R² in the definition of -NR¹R² are each selected independently from hydrogen, formyl, phenyl, benzyl, benzoyl, (C₃-C₈)cycloalkyl, (C₃-C₈)cycloalkenyl, (C₁-C₄)alkyl, (C₁-C₄)perfluoroalkyl, (C₁-C₁₀)alkoxycarbonyl, (C₁-C₆)acyl, (C₁-C₆)perfluoroacyl, aminocarbonyl, (C₁-C₁₀)alkylaminocarbonyl, di(C₁-C₁₀)alkylaminocarbonyl, aminosulfonyl, (C₁-C₄)alkylaminosulfonyl, di(C₁-C4)alkylaminosulfonyl, (C₁-C₄)perfluoroalkylaminosulfonyl, di(C₁-C₄)perfluoroalkylaminosulfonyl, (C₁-C₄)alkylsulfonyl, and (C₁-C₄)perfuoroalkylsulfonyl,
or where R¹ and R², taken together with the nitrogen atom to which they are attached, form a saturated, partially-saturated or aromatic heterocyclic ring, wherein said heterocyclic ring contains a total of from 3 to 14 ring atoms and incorporates optionally an additional 1 to 4 ring heteroatoms selected independently from oxygen, nitrogen and sulfur, wherein said heterocyclic ring may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, and (C₁-C₁₀)acyloxy,
where R³ in the definition of -OCOR³ is selected from -NR¹R², phenyl, (C₁-C₁₀)alkyl, (C₁-C₄)perfluoroalkyl, (C₁-C₆)alkoxy and (C₁-C₆)perfluoroalkoxy,
(2) (C₃-C₈)cycloalkyl or (C₃-C₈)cycloalkenyl wherein each of said (C₃-C₈)cycloalkyl and (C₃-C₈)cycloalkenyl may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfuoroacyl, (C₁-C₁₀)acylamino, (C₁-C₁₀)perfuoroacylamino, (C₁-C₁₀)acyloxy, and
(3) a saturated, partially-saturated or aromatic heterocyclic ring containing a total of from 3 to 14 ring atoms, wherein said heterocyclic ring incorporates a total of from 1 to 4 ring heteroatoms selected independently from oxygen, nitrogen and sulfur, wherein said heterocyclic ring may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfuoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfuoroacyl, (C₁-C₁₀)acylamino, (C₁-C₁₀)perfluoroacylamino, (C₁-C₁₀)acyloxy;
(e) (C₃-C₈)cycloalkyl or (C₃-C₈)cycloalkenyl wherein each of said (C₃-C₈)cycloalkyl and (C₃-C₈)cycloalkenyl may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)Perfuoroacyl, (C₁-C₁₀)acylamino, (C₁-C₁₀)perfuoroacylamino, (C₁-C₁₀)acyloxy; and
(f) -(CH₂)ₙCOR⁴, where R⁴ in the definition of -(CH₂)ₙCOR⁴ is selected from hydroxy, phenyl, -NR¹R², (C₁-C₄)alkyl, (C₁-C₄)perfluoroalkyl, (C₁-C₄)alkoxy, (C₁-C₄)perfluoroalkoxy, (C₃-C₈)cycloalkyl, and (C₃-C₈)cycloalkenyl, where n is an integer from 1 to 4;
(ii) the compound 9-[4-[4-(2,3-dihydro-1-oxo-1H-isoindol-2-yl)-1-piperidinyl]butyl]-N-propyl-9H-fluorene-9-carboxamide, and the pharmaceutically acceptable salts thereof;
(iii) the compound 2-[1-(3,3-diphenylpropyl)-4-piperidinyl)-2,3-dihydro-1H-isoindol-1-one, and the pharmaceutically acceptable salts thereof; and
(iv) the compound 9-[4-(4-[2-(4-trifluoromethylphenyl)benzoylamino]piperidin-1-yl)butyl]-N-2,2,2-trifluoroethyl)-9H-fluorene-9-carboxamide, and the pharmaceutically acceptable salts thereof; and
said anti-obesity agent is selected from the group consisting of a β₃-adrenergic receptor agonist, a cholecystokinin-A agonist, a monoamine reuptake inhibitor, a sympathomimetic agent, a serotoninergic agent, a dopamine agonist, a melanocyte-stimulating hormone receptor agonist or mimetic, a melanocyte-stimulating hormone receptor analog, a cannabinoid receptor antagonist, a melanin concentrating hormone antagonist, leptin, a leptin analog, a leptin receptor agonist, a galanin antagonist, a lipase inhibitor, a bombesin agonist, a Neuropeptide-Y antagonist, a thyromimetic agent, dehydroepiandrosterone or an analog thereof, a glucocorticoid receptor agonist or antagonist, an orexin receptor antagonist, a urocortin binding protein antagonist, a glucagon-like peptide-1 receptor agonist, a ciliary neurotrophic factor, and a human agouti-related protein antagonist.

8. Use according to claim 7, wherein said apolipoprotein B secretion/microsomal triglyceride transfer protein inhibitor is a compound selected from the group consisting of:
4'-trifluoromethyl-biphenyl-2-carboxylic acid-(2-butyl-1,2,3,4-tetrahydroisoquinolin-6-yl)-amide, the hydrates thereof, and the pharmaceutically acceptable salts of said compound and said hydrates;
4'-trifluoromethyl-biphenyl-2-carboxylic acid-[2-(2-acetylaminoethyl)-1,2,3,4-tetrahydroisoquinolin-6-yl amide, the hydrates thereof, and the pharmaceutically acceptable salts of said compound and said hydrates;
9-[4-[4-(2,3-dihydro-1 -oxo-1 H-isoindol-2-yl)-1-piperidinyl]butyl]-N-propyl-9H-fluorene-9-carboxamide, and the pharmaceutically acceptable salts thereof;
2-[1-(3,3-diphenylpropyl)-4-piperidinyl]-2,3-dihydro-1H-isoindol-1-one, and the pharmaceutically acceptable salts thereof; and
9-[4-(4-[2-(4-trifluoromethylphenyl)benzoylamino]piperidin-1-yl)butyl]-N-2,2,2-trifluoroethyl)-9H-fluorene-9-carboxamide, and the pharmaceutically acceptable salts thereof; and
said anti-obesity agent is selected from the group consisting of sibutramine, orlistat, fenfluramine, dexfenfluramine, bromocriptine, phentermine, ephedrine, leptin, phenylpropanolamine, pseudoephedrine, {4-[2-(2-[6-aminopyridin-3-yl]-2(R)-hydroxyethylamino)ethoxy]phenyl}acetic acid, {4-[2-(2-[6-aminopyridin-3-yl]-2(R)-hydroxyethylamino)ethoxy]phenyl}benzoic acid, {4-[2-(2-[6-aminopyridin-3-yl]-2(R)-hydroxyethylamino)ethoxy]phenyl}propionic acid, and {4-[2-(2-[6-aminopyridin-3-yl]-2(R)-hydroxyethylamino)ethoxy]phenoxy}acetic acid.

9. A pharmaceutical composition comprising amounts of an apolipoprotein B secretion/microsomal triglyceride transfer protein inhibitor, and an anti-obesity agent.

10. A composition according to claim 9 further comprising a pharmaceutically acceptable carrier, vehicle or diluent.

11. A composition according to claim 9, wherein said apolipoprotein B secretion/microsomal triglyceride transfer protein inhibitor is a compound selected from the group consisting of:
(i) a compound of formula (I) 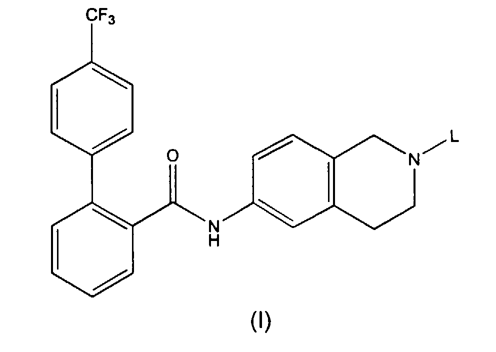 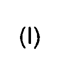 the stereoisomers and hydrates thereof, and the pharmaceutically acceptable salts of said compounds, stereoisomers and hydrates, wherein L represents:
X-Y-Z, wherein:
X is a moiety selected from the group consisting of CH₂, CO, CS, or SO₂;
Y is a moiety selected from the group consisting of a direct link, aliphatic hydrocarbylene radicals having up to 20 carbon atoms, which radical may be monosubstituted by hydroxy, (C₁-C₁₀)alkoxy, (C₁-C₁₀)acyl, (C₁-C₁₀)acyloxy, or (C₆-C₁₀)aryl, NH, and O, provided that if X is CH₂, Y is a direct link; and
Z is a moiety selected from the group consisting of:
(1) hydrogen, halogen, cyano,
(2) hydroxy, (C₁-C₁₀)alkoxy, (C₁-C₁₀)alkylthio, (C₁-C₁₀)acyl, thiophenylcarbonyl, (C₁-C₁₀)alkoxycarbonyl,
(3) (C₁-C₁₀)alkylamino, di(C₁-C₁₀)alkylamino, (C₆-C₁₀)aryl(C₁-C₁₀)alkylamino, provided that Y is not O or NH,
(4) unsubstituted vinyl, (C₆-C₁₀)aryl, (C₃-C₈)cycloalkyl and fused benz derivatives thereof, (C₇-C₁₀)polycycloalkyl, (C₄-C₈)cycloalkenyl, (C₇-C₁₀)polycycloalkenyl,
(5) (C₆-C₁₀)aryloxy, (C₆-C₁₀)arylthio, (C₆-C₁₀)aryl(C₁-C₁₀)alkoxy, (C₆-C₁₀)aryl(C₁-C₁₀)alkylthio, (C₃-C₈)cycloalkyloxy, (C₄-C₈)cycloalkenyloxy,
(6) heterocyclyl selected from the group consisting of monocyclic radicals and fused polycyclic radicals, wherein said radicals contain a total of from 5 to 14 ring atoms, wherein said radicals contain a total of from 1 to 4 ring heteroatoms independently selected from oxygen, nitrogen, and sulfur, and wherein the individual rings of said radicals may be independently saturated, partially unsaturated, or aromatic, provided that if X is CH₂, Z is H or is selected from groups (4) and (6),
wherein, when Z contains one or more rings, said rings may each independently bear 0 to 4 substituents independently selected from halo, hydroxy, cyano, nitro, oxo, thioxo, aminosulfonyl, phenyl, phenoxy, phenylthio, halophenylthio, benzyl, benzyloxy, (C₁-C₁₀)alkyl, (C₁-C₁₀)alkoxy, (C₁-C₁₀)alkoxycarbonyl, (C₁-C₁₀)alkylthio, (C₁-C₁₀)alkylamino, (C₁-C₁₀)alkylaminocarbonyl, di(C₁-C₁₀)alkylamino, di(C₁-C₁₀)alkylaminocarbonyl, di(C₁-C₁₀)alkylamino(C₁-C₁₀)alkoxy, (C₁-C₃)perfluoroalkyl, (C₁-C₃)perfluoroalkoxy, (C₁-C₁₀)acyl, (C₁-C₁₀)acyloxy, (C₁-C₁₀)acyloxy(C₁-C₁₀)alkyl, and pyrrolidinyl; or
G, wherein G is selected from the group consisting of:
(a) a phenyl or heterocyclic ring wherein said heterocyclic ring contains a total of from 3 to 14 ring atoms, wherein said heterocyclic ring incorporates a total of from 1 to 4 ring heteroatoms selected independently from oxygen, nitrogen, and sulfur, wherein the individual rings of said heterocyclic ring may be independently saturated, partially saturated or aromatic, and wherein each of said phenyl or heterocyclic rings may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acyloxy, (C₁-C₆)acylamino and (C₁-C₆)perfuoroacylamino;
(b) -CH₂CN,
(c) 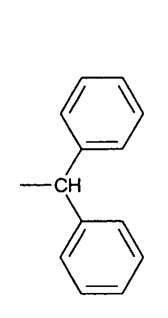
(d) (C₂-C₁₂)alkyl or (C₂-C₁₂)perfluoroalkyl wherein each of said (C₂-C₁₂)alkyl and (C₂-C₁₂)perfluoroalkyl is substituted optionally with from 1-3 substituents selected independently from:
(1) phenyl, halogen, nitro, cyano, hydroxy, -NR¹R², -OCOR³, (C₁-C₄)alkoxy, (C₁-C₄)perfluoroalkoxy, (C₁-C₄)thioalkoxy or (C₁-C₄)perfuorothioalkoxy,
where R¹ and R² in the definition of -NR¹R² are each selected independently from hydrogen, formyl, phenyl, benzyl, benzoyl, (C₃-C₈)cycloalkyl, (C₃-C₈)cycloalkenyl, (C₁-C₄)alkyl, (C₁-C₄)perfluoroalkyl, (C₁-C₁₀)alkoxycarbonyl, (C₁-C₆)acyl, (C₁-C₆)perfluoroacyl, aminocarbonyl, (C₁-C₁₀)alkylaminocarbonyl, di(C₁-C₁₀)alkylaminocarbonyl, aminosulfonyl, (C₁-C₄)alkylaminosulfonyl, di(C₁-C₄)alkylaminosulfonyl, (C₁-C₄)perfluoroalkylaminosulfonyl, di(C₁-C₄)perfuoroalkylaminosulfonyl, (C₁-C₄)alkylsulfonyl, and (C₁-C₄)perfuoroalkylsulfonyl,
or where R¹ and R², taken together with the nitrogen atom to which they are attached, form a saturated, partially-saturated or aromatic heterocyclic ring, wherein said heterocyclic ring contains a total of from 3 to 14 ring atoms and incorporates optionally an additional 1 to 4 ring heteroatoms selected independently from oxygen, nitrogen and sulfur, wherein said heterocyclic ring may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfuoroacyl, (C₁-C₁₀)acylamino, and (C₁-C₁₀)acyloxy,
where R³ in the definition of -OCOR³ is selected from -NR¹R², phenyl, (C₁-C₁₀)alkyl, (C₁-C₄)perfluoroalkyl, (C₁-C₆)alkoxy and (C₁-C₆)perfluoroalkoxy,
(2) (C₃-C₈)cycloalkyl or (C₃-C₈)cycloalkenyl wherein each of said (C₃-C₈)cycloalkyl and (C₃-C₈)cycloalkenyl may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfluoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfuoroacyl, (C₁-C₁₀)acylamino, (C₁-C₁₀)perfluoroacylamino, (C₁-C₁₀)acyloxy, and
(3) a saturated, partially-saturated or aromatic heterocyclic ring containing a total of from 3 to 14 ring atoms, wherein said heterocyclic ring incorporates a total of from 1 to 4 ring heteroatoms selected independently from oxygen, nitrogen and sulfur, wherein said heterocyclic ring may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfuoroalkyl, (C₁-C₁₀)alkoxy, (C₁-C₄)perfuoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfluoroacyl, (C₁-C₁₀)acylamino, (C₁-C₁₀)perfluoroacylamino, (C₁-C₁₀)acyloxy;
(e) (C₃-C₈)cycloalkyl or (C₃-C₈)cycloalkenyl wherein each of said (C₃-C₈)cycloalkyl and (C₃-C₈)cycloalkenyl may have optionally from 1 to 4 substituents selected independently from halogen, hydroxy, cyano, nitro, oxo, thioxo, aminosulfonyl, phenyl, phenoxy, phenylthio, benzyl, benzoyl, benzyloxy, (C₁-C₁₀)alkyl, (C₁-C₄)perfluoroalkyl, (C₁-C₁₀)alkoxy, (C₁-C₄)perfuoroalkoxy, (C₁-C₁₀)alkoxycarbonyl, (C₁-C₁₀)alkylthio, (C₁-C₁₀)alkylamino, di(C₁-C₁₀)alkylamino, (C₁-C₁₀)alkylaminocarbonyl, di(C₁-C₁₀)alkylaminocarbonyl, (C₁-C₁₀)acyl, (C₁-C₁₀)perfuoroacyl, (C₁-C₁₀)acylamino, (C₁-C₁₀)perfuoroacylamino, (C₁-C₁₀)acyloxy; and
(f) -(CH₂)ₙCOR⁴, where R⁴ in the definition of -(CH₂)ₙCOR⁴ is selected from hydroxy, phenyl, -NR¹R², (C₁-C₄)alkyl, (C₁-C₄)perfluoroalkyl, (C₁-C₄)alkoxy, (C₁-C₄)perfluoroalkoxy, (C₃-C₈)cycloalkyl, and (C₃-C₈)cycloalkenyl, where n is an integer from 1 to 4;
(ii) the compound 9-[4-[4-(2,3-dihydro-1-oxo-1H-isoindol-2-yl)-1-piperidinyl]butyl]-N-propyl-9H-fluorene-9-carboxamide, and the pharmaceutically acceptable salts thereof;
(iii) the compound 2-[1-(3,3-diphenylpropyl)-4-piperidinyl]-2,3-dihydro-1H-isoindol-1-one, and the pharmaceutically acceptable salts thereof; and
(iv) the compound 9-[4-(4-[2-(4-trifluoromethylphenyl)benzoylamino]piperidin-1-yl)butyl]-N-2,2,2-trifluoroethyl)-9H-fluorene-9-carboxamide; and the pharmaceutically acceptable salts thereof; and
said anti-obesity agent is selected from the group consisting of a β₃-adrenergic receptor agonist, a cholecystokinin-A agonist, a monoamine reuptake inhibitor, a sympathomimetic agent, a serotoninergic agent, a dopamine agonist, a melanocyte-stimulating hormone receptor agonist or mimetic, a melanocyte-stimulating hormone receptor analog, a cannabinoid receptor antagonist, a melanin concentrating hormone antagonist, leptin, a leptin analog, a leptin receptor agonist, a galanin antagonist, a lipase inhibitor, a bombesin agonist, a Neuropeptide-Y antagonist, a thyromimetic agent, dehydroepiandrosterone or an analog thereof, a glucocorticoid receptor agonist or antagonist, an orexin receptor antagonist, a urocortin binding protein antagonist, a glucagon-like peptide-1 receptor agonist, a ciliary neurotrophic factor, and a human agouti-related protein antagonist.

12. A composition according to claim 11, wherein said apo B secretion/microsomal triglyceride transfer protein inhibitor is a compound selected from the group consisting of:
4'-trifluoromethyl-biphenyl-2-carboxylic acid-(2-butyl-1,2,3,4-tetrahydroisoquinolin-6-yl)-amide, the hydrates thereof, and the pharmaceutically acceptable salts of said compound and said hydrates;
4'-trifluoromethyl-biphenyl-2-carboxylic acid-[2-(2-acetylaminoethyl)-1,2,3,4-tetrahydroisoquinolin-6-yl amide, the hydrates thereof, and the pharmaceutically acceptable salts of said compound and said hydrates;
9-[4-[4-(2,3-dihydro-1-oxo-1H-isoindol-2-yl)-1-piperidinyl]butyl]-N-propyl-9H-fluorene-9-carboxamide, and the pharmaceutically acceptable salts thereof;
2-[1-(3,3-diphenylpropyl)-4-piperidinyl]-2,3-dihydro-1H-isoindol-1-one, and the pharmaceutically acceptable salts thereof; and
9-[4-(4-[2-(4-trifluoromethylphenyl)benzoylamino]piperidin-1-yl)butyl]-N-2,2,2-trifluoroethyl)-9H-fluorene-9-carboxamide; and the pharmaceutically acceptable salts thereof; and
said anti-obesity agent is a compound selected from the group consisting of sibutramine, orlistat, fenfluramine, dexfenfluramine, bromocriptine, phentermine, ephedrine, leptin, phenylpropanolamine, pseudoephedrine, {4-[2-(2-[6-aminopyridin-3-yl]-2(R)-hydroxyethylamino)ethoxy]phenyl}acetic acid, {4-[2-(2-[6-aminopyridin-3-yl]-2(R)-hydroxyethylamino)ethoxy]phenyl}benzoic acid, {4-[2-(2-[6-aminopyridin-3-yl]-2(R)-hydroxyethylamino)ethoxy]phenyl}propionic acid and {4-[2-(2-[6-aminopyridin-3-yl]-2(R)-hydroxyethylamino)ethoxy]phenoxy}acetic acid.

13. Use of the composition of any one of claims 9 to 12 in the manufacture of a medicament for treating obesity.

14. A kit comprising an amount of an apolipoprotein B secretion/microsomal triglyceride transfer protein inhibitor and a pharmaceutically acceptable carrier, vehicle or diluent in a first unit dosage form; an amount of an anti-obesity agent and a pharmaceutically acceptable carrier, vehicle or diluent in a second unit dosage form; and a container.

15. Products containing:
(a) an amount of an apolipoprotein B secretion/microsomal triglyceride transfer protein inhibitor;
(b) an amount of an anti-obesity agent;
as a combined preparation for simultaneous, separate or sequential use in treating obesity.
